Europäisches Patentamt

European Patent Office

Office européen des brevets

(11) Veröffentlichungsnummer: **0 508 216 B1**

## (12) EUROPÄISCHE PATENTSCHRIFT

(45) Veröffentlichungstag der Patentschrift: **05.10.94**

(51) Int. Cl.⁵: **C07C 263/18**, C07C 265/14

(21) Anmeldenummer: **92105102.5**

(22) Anmeldetag: **25.03.92**

(54) **Verfahren zur Konditionierung und/oder Reinigung von organischen Isocyanaten.**

(30) Priorität: **06.04.91 DE 4111212**

(43) Veröffentlichungstag der Anmeldung:
**14.10.92 Patentblatt 92/42**

(45) Bekanntmachung des Hinweises auf die
Patenterteilung:
**05.10.94 Patentblatt 94/40**

(84) Benannte Vertragsstaaten:
**AT BE CH DE ES FR GB IT LI NL SE**

(56) Entgegenhaltungen:
**EP-A- 0 341 516**
**CH-A- 464 180**
**DE-A- 2 837 770**
**US-A- 3 759 971**

**PATENT ABSTRACTS OF JAPAN, vol. 6, no.
165 (C-121)(1043), 28. August 1982; & JP-
A-57082358**

**CHEMICAL ABSTRACTS, vol. 114, no. 14, 8.
April 1991, Columbus, Ohio, US; abstract no.
124644E, Seite 115 ;Spalte 1; & JP-A-2140277**

(73) Patentinhaber: **BAYER AG**

**D-51368 Leverkusen (DE)**

(72) Erfinder: **Scholl, Hans-Joachim, Dr.
Am Feldrain 5
W-5000 Köln 80 (DE)**

**Beschreibung**

Die Erfindung betrifft ein neues Verfahren zur Konditionierung und/oder Reinigung von organischen Isocyanaten durch Vermischen der Isocyanate mit bestimmten silylierten Säuren und gegebenenfalls anschließender destillativer Aufarbeitung der so erhaltenen Gemische.

Herstellungsbedingte Verunreinigungen wechselnder Art und Menge in Isocyanaten sind Anlaß für Aktivitätsschwankungen und Farbprobleme, deren Auswirkungen bis in entsprechende Folgeprodukte reichen. Eine Limitierung, d.h. eine Verengung des Spezifikationsrahmens, insbesondere bei technischen Isocyanaten, ist ein bedeutsames Ziel zu ihrer verbesserten und damit wirtschaftlicheren Handhabung (vgl. CH-A-464180, EP-A-0341516, JP-A-57082358).

Es war daher die der Erfindung zugrundeliegende Aufgabe, ein neues Verfahren zur Konditionierung und/oder Reinigung von organischen Isocyanaten zur Verfügung zu stellen, welches die angesprochenen Mängel beheben hilft.

Diese Aufgabe konnte mit dem nachstehend näher beschriebenen erfindungsgemäßen Verfahren gelöst werden. Das Prinzip des erfindungsgemäßen Verfahrens besteht darin, den zu behandelnden technischen Isocyanaten eine geringe Menge von bestimmten, nachstehend näher beschriebenen silylierten Säuren zuzusetzen und die so erhaltenen Gemische gegebenenfalls anschließend destillativ aufzuarbeiten.

Gegenstand der Erfindung ist ein Verfahren zur Konditionierung und/oder Reinigung von organischen Isocyanaten, dadurch gekennzeichnet, daß man organische Isocyanate bei 20 bis 150 °C mit 0,001 bis 1 Mol-%, bezogen auf die Menge des Isocyanats, einer silylierten Säure der Formel

$$X\text{-}[Si(CH_3)3]_n$$

vermischt und gegebenenfalls die so erhaltene Mischung nach einer Verweildauer von mindestens 5 Minuten einer destillativen Aufarbeitung unterzieht,
wobei

X für den neutralen Säurerest steht, wie er durch Entfernung der aciden Wasserstoffatome aus einer n-basischen Säure mit einem pKa-Wert von maximal 3 erhalten wird, wobei Halogenwasserstoffsäuren ausgenommen sind, und

n für eine ganze Zahl von 1 bis 3 steht.

Als Ausgangsmaterialien für das erfindungsgemäße Verfahren können beliebige organische Isocyanate eingesetzt werden. Vorzugsweise wird das erfindungsgemäße Verfahren jedoch zur Konditionierung und/oder Reinigung von organischen Diisocyanaten der in der Polyurethanchemie eingesetzten Art verwendet,

Hierzu gehören insbesondere aliphatische Diisocyanate wie 1,6-Diisocyanatohexan (HDI), 1-Isocyanato-3,3,5-trimethyl-5-isocyanatomethyl-cyclohexan (IPDI), 4,4'-Diisocyanato-dicyclohexylmethan (HMDI) und beliebige Gemische derartiger aliphatischer Diisocyanate. Auch aromatische Diisocyanate wie 2,4- und/oder 2,6-Diisocyanatotoluol (TDI), 2,2'-, 2,4'- und/oder 4,4'-Diisocyanatodiphenylmethan (MDI) bzw. beliebige Gemische derartiger aromatischer Diisocyanate können erfindungsgemäß als Ausgangsmaterial eingesetzt werden.

Auch beliebige modifizierte Polyisocyanate können als erfindungsgemäße Ausgangsmaterialien verwendet werden, Von besonderem Interesse sind in diesem Zusammenhang Reaktionsgemische wie sie bei der Trimerisierung eines Teils der Isocyanatgruppen von HDI, IPDI oder Gemischen aus HDI und IPDI zwecks Herstellung der entsprechenden Isocyanuratgruppen aufweisenden Polyisocyanate erhalten werden, und die im wesentlichen aus den genannten Ausgangsdiisocyanaten und den entstehenden Isocyanuratgruppen aufweisenden Polyisocyanaten bestehen, Im Falle der Verwendung derartiger Gemische als Ausgangspolyisocyanate für das erfindungsgemäße Verfahren erfolgt der Zusatz der erfindungswesentlichen Zusatzmittel vorzugsweise zu dem Zeitpunkt, an dem die Trimerisierungsreaktion abgebrochen werden soll, da die erfindungswesentlichen Zusatzmittel in vielen Fällen auch die Funktion eines Katalysatorengifts erfüllen und damit als Stopper für die Trimerisierungsreaktion wirken. Besonders hochwertige Trimerisierungsprodukte der genannten aliphatischen Diisocyanate werden dann erhalten, wenn man als Ausgangsdiisocyanate erfindungsgemäß vorbehandelte Diisocyanate verwendet und anschließend die Trimerisierungsprodukte wie soeben beschrieben behandelt.

Bei den erfindungswesentlichen Zusatzmitteln handelt es sich um silylierte Säuren der Formel

$$X\text{-}[Si(CH_3)_3]_n.$$

In dieser Formel haben X und n die bereits obengenannte Bedeutung. Vorzugsweise steht

X    für den neutralen Säurerest einer n acide Wasserstoffatome aufweisenden, Sauerstoff enthaltenden Säure eines maximalen pKa-Werts von 2.

Geeignet sind beispielsweise entsprechende silylierte Sulfonsäuren wie Trifluormethansulfonsäuretrimethylsilylester oder Methansulfonsäuretrimethylsilylester, silylierte Ester von Säuren des Phosphors wie Phosphorsäuretris(trimethylsilylester) oder Phosphorsäurediethylester-trimethylsilylester.

Die beispielhaft genannten, erfindungswesentlichen Zusatzmittel werden den Ausgangsisocyanaten in einer Menge von 0,001 bis 1,0, vorzugsweise 0,001 bis 0,1 Mol-%, bezogen auf Isocyanat, zugesetzt. Ihre optimale Menge kann leicht durch einen orientierenden Vorversuch ermittelt werden, Die Zugabe erfolgt innerhalb des Temperaturbereichs von 20 bis 150, vorzugsweise 50 bis 120° C.

Vorzugsweise wird das so erhaltene Gemisch nach einem Zeitraum von mindestens 5, vorzugsweise von mindestens 30 Minuten einer destillativen Aufarbeitung unterzogen. Hierunter ist im Falle der Verwendung von destillierbaren Ausgangsisocyanaten deren destillative Reindarstellung, beispielsweise im Dünnschichtverdampfer zu verstehen, während die destillative Aufarbeitung im Falle der Verwendung von Gemischen aus monomeren Diisocyanaten und nicht destillierbaren, Isocyanuratgruppen aufweisenden Polyisocyanaten in der destillativen Entfernung der flüchtigen Ausgangsdiisocyanate besteht, Auch diese destillative Aufarbeitung erfolgt vorzugsweise unter Verwendung von Dünnschichtverdampfern, wobei die Destillationsbedingungen so gewählt werden, daß die das Verfahrensprodukt darstellenden Destillationsrückstände einen Gehalt an monomeren Ausgangsdiisocyanaten von maximal 2,0, vorzugsweise von maximal 0,5 Gew.-% aufweisen.

Die erfindungsgemäß konditionierten Isocyanate weisen vergleichsweise verringerte Aktivitätsschwankungen auf und enthalten reduzierte Spurenanteile an farbgebenden Verunreinigungen. Dies läßt sich besonders einfach anhand von Folgereaktionen wie beispielsweise der Herstellung von Isocyanuratgruppen aufweisenden Polyisocyanaten unter Trimerisierung eines Teils der Isocyanatgruppen von einfachen, erfindungsgemäß behandelten Diisocyanaten zeigen.

Im Zusammenhang mit der Herstellung von Isocyanuratgruppen aufweisenden Lackpolyisocyanaten aus aliphatischen Ausgangsdiisocyanaten der beispielhaft genannten Art ist die erfindungsgemäße Konditionierung im zweifacher Hinsicht von Interesse. Einerseits ist es aus den soeben genannten Gründen vorteilhaft, als Ausgangsmaterial zur Herstellung dieser Lackpolyisocyanate erfindungsgemäß vorbehandelte Diisocyanate einzusetzen, und andererseits bewirkt auch die Zugabe der erfindungswesentlichen Zusatzmittel zu dem bei der Trimerisierungsreaktion anfallenden Reaktionsgemisch vor dessen destillativer Aufarbeitung eine Konditionierung und insbesondere Verbesserung der Farbwerte und zwar nicht nur der destillativ rückgewonnenen, überschüssigen Ausgangsdiisocyanate, die für eine weitere Umsetzung eingesetzt werden, sondern auch der Verfahrensprodukte, d.h. der bei der destillativen Aufarbeitung anfallenden Destillationsrückstände.

Dies gilt insbesondere bei der an sich bekannten Trimerisierung von aliphatischen Diisocyanaten, insbesondere von HDI unter Verwendung von quartären Ammoniumhydroxiden oder, besonders bevorzugt, quartären Ammoniumfluoriden als Trimerisierungskatalysator. Im Falle der Verwendung dieser Katalysatoren erfüllen die erfindungswesentlichen Zusatzmittel im übrigen die Funktion eines Katalysatorengifts, so daß die erfindungsgemäße Konditionierung mit der Abstoppung der Trimerisierungsreaktion beim jeweils gewünschten Trimerisierungsgrad einhergeht.

Beispiele

Ausgangsmaterialien:

Quartäres Ammoniumsalz I (nach DE 38 27 596)

2,9 g Kaliumfluorid in 50 g Ethanol werden unter Rühren bei Raumtemperatur mit 20 g handelsüblichem quartärem Ammoniumchlorid bestehend im wesentlichen aus N-Methyl-N,N,N-trialkylammoniumchlorid mit $C_8$-$C_{10}$-Alkylresten (®Aliquat 336 der Firma Fluka GmbH, D 7910 Neu-Ulm), welches in 60 g Ethanol gelöst vorliegt, versetzt. Nach 60 Minuten filtriert man von unlöslichen Bestandteilen ab, fügt dem Filtrat 476 g 2-Ethylhexanol-1 hinzu und trennt von Ethanol im Vakuum ab, Auf diese Weise entsteht eine Lösung eines quartären Ammoniumfluorids in Ethylhexanol-1 mit einem Fluoridgehalt von 0,07 mmol/g.

Quartäres Ammoniumsalz II (nach DE 38 06 276)

0,5 Gew.-% N,N,N-Trimethyl-N-benzyl-ammoniumhydroxid in 2-Ethylhexandiol-1,3,

Beispiel 1

Umsetzung von HDI mit Trifluormethansulfonsäuretrimethylsilylester (TMS-triflat).
3528 g (21 Mol) technisches HDI werden mit 7,3 g einer 2,3 %igen TMS-triflat-Lösung in HDI 1 Stunde bei 100°C gerührt. Das HDI wurde nachfolgend durch Dünnschichtdestillation rückgewonnen.

Beispiel 2

Umsetzung von HDI mit Phosphorsäuretris(trimethylsilylester).
3360 g (20 Mol) technisches HDI werden mit 7 g einer 1 %igen Phosphorsäuretris(trimethylsilylester)-Lösung in HDI 1 Stunde bei 100°C gerührt. Das HDI wurde nachfolgend durch Dünnschichtdestillation rückgewonnen.

Beispiel 3

Teiltrimerisierung der vorbehandelten Diisocyanate aus Beispiel 1 und 2 im Vergleich zu unbehandeltem HDI aus gleicher Herstellung.
Die Beispiele dokumentieren die erfindungsgemäßen Vorteile: Aktivitätsverbesserung und Verbesserung der Farbqualität.
Jeweils 840 g (5 Mol) HDI wurden bei 55°C für 7 Stunden mit Ammoniumsalz-I-Katalyse einer Teiltrimerisierung unterworfen. Nach Abstoppen mit dem jeweiligen Katalysatorgift wurden die Produkte vom nicht umgesetzten HDI durch Dünnschichtdestillation befreit. In Tabelle 1 sind die wesentlichen Einzeldaten zusammengestellt.

## Tabelle 1

|  | Beispiel 3a | Beispiel 3b | Vergleich Beispiel 3c |
|---|---|---|---|
| Einsatzprodukt | HDI (Beispiel 1) | HDI (Beispiel 2) | HDI-Betriebsware |
| Katalysator | 5 g | 4 g | 5 g |
| Beginn: $n_D^{23}$ | 1.4520 | 1.4520 | 1.4520 |
| Ende: $n_D^{23}$ | 1.4622 | 1.4650 | 1.4598 |
| Katalysatorgift[1]) | TMS-triflat | Phosphorsäuretris-(trimethylsilylester) | Phosphorsäuredibutylester |
| **Endprodukte:** | | | |
| - Ausbeute | 22,5 % | 27,5 % | 17,7 % |
| - NCO | 23,1 % | 22,8 % | 22,9 % |
| - Farbqualität | gut: farblos | gut: farblos | schlecht: gelb |

Die eindeutige Überlegenheit der erfindungsgemäßen Verfahrensprodukte in Bezug auf höheren Umsatz (Aktivitätsverbesserung) und verbesserte Farbqualität ist augenscheinlich.

[1]) Menge: jeweils 30 Mol-%, bezogen auf eingesetzten Katalysator

Beispiel 4

Teiltrimerisierung des erfindungsgemäß vorbehandelten Diisocyanats aus Beispiel 2 im Vergleich zu unbehandeltem HDI aus gleicher Herstellung unter Verwendung von quartärem Ammoniumsalz II als Trimerisierungskatalysator. Die Beispiele dokumentieren die erfindungsgemäßen Vorteile: Aktivitätsverbesserung und Verbesserung der Farbqualität.

Jeweils 840 g (5 Mol) HDI wurden bei 55 °C für 7 Stunden mit Ammoniumsalz-II-Katalyse einer Teiltrimerisierung unterworfen. Nach Abstoppen mit dem jeweiligen Katalysatorgift wurden die Produkte vom nicht umgesetzten HDI durch Dünnschichtdestillation befreit. In Tabelle 2 sind die wesentlichen Einzeldaten zusammengestellt.

Tabelle 2

| | Beispiel 4a | Vergleich Beispiel 4b |
|---|---|---|
| Einsatzprodukt | HDI (Beispiel 2) | HDI (nicht vorbehandelt) |
| Katalysator | 8 g | 9 g |
| Beginn: $n_D^{23}$ | 1.4520 | 1.4520 |
| Ende: $n_D^{23}$ | 1.4643 | 1.4566 |
| Katalysatorgift[1] | Phosphorsäuretris-(trimethylsilylester) | Phosphorsäuredi-butylester |
| Endprodukte: | | |
| - Ausbeute | 26,9 % | 11,6 % |
| - NCO | 22,1 % | 21,0 % |
| - Farbqualität | gut: farblos | schlecht: gelb |

Die Überlegenheit der erfindungsgemäßen Verfahrensprodukte in Bezug auf höheren Umsatz (Aktivitätsverbesserung) und verbesserte Farbqualität ist augenscheinlich.

[1] Menge: jeweils 40 Mol-%, bezogen auf eingesetzten Katalysator

Beispiel 5

Umsetzung von HDI/IPDI (4:1 molar) mit Trifluormethansulfonsäuretrimethylsilylester (TMS-triflat):
Eine Mischung von 672 g (4 Mol) technischem HDI und 222 g (1 Mol) technischem IPDI wurde mit 3,4 g einer 2,3 %igen TMS-triflat-Lösung in HDI während eines Zeitraums von 0,5 Stunden auf 100 °C erwärmt und durch Dünnschichtdestillation rückgewonnen.

Beispiel 6

Teiltrimerisierung des erfindungsgemäß vorbehandelten HDI/IPDI-Gemisches aus Beispiel 5 im Vergleich zum unbehandelter HDI/IPDI-Gemisch gleicher molarer Zusammensetzung aus gleicher Herstellung: Jeweils 447 g (2 Mol HDI, 0,5 Mol IPDI) Gemisch wurden bei 55 °C für 7 Stunden mit Ammoniumsalz-I-Katalyse einer Teiltrimerisierung unterworfen. Nach Abstoppen mit dem jeweiligen Katalysatorgift wurden die Produkte vom nicht umgesetzten HDI/IPDI durch Dünnmschichtdestillation befreit. In Tabelle 3 sind die wesentlichen Einzeldaten zusammengestellt.

**Tabelle 3**

|                              | Beispiel 6a            | Vergleich<br>Beispiel 6b        |
|------------------------------|------------------------|---------------------------------|
| Einsatzprodukt               | HDI/IPDI (Beispiel 5)  | HDI/IPDI (ohne Vorbehandlung)   |
| Katalysator                  | 5,5 g                  | 5,5 g                           |
| Beginn: $n_D^{23}$           | 1.4595                 | 1.4595                          |
| Ende: $n_D^{23}$             | 1.4679                 | 1.4643                          |
| Katalysatorgift[1])          | TMS-triflat            | Phosphorsäuredi-butylester      |
| Endprodukte:                 |                        |                                 |
| - Ausbeute                   | 16,8 %                 | 9,5 %                           |
| - NCO                        | 21,7 %                 | 21,6 %                          |
| - Farbqualität               | gut: hellgelb          | schlecht: gelb                  |

Die eindeutige Überlegenheit der erfindungsgemäßen Verfahrensprodukte in Bezug auf höheren Umsatz (Aktivitätsverbesserung) und verbesserte Farbqualität ist augenscheinlich.

[1]) Menge: jeweils 20 Mol-%, bezogen auf eingesetzten Katalysator

Vergleichsbeispiele (zum Beleg verbesserter Stopperwirksamkeit der erfindungswesentlichen Silylverbindungen im Vergleich zu Phosphorsäuredibutylester)

A) Teiltrimerisierung von HDI

3528 g (21 Mol) technisches HDI werden unter $N_2$/Rühren auf 55°C erwärmt und mit 14 g quartärem Ammoniumsalz I versetzt. Nach 4 Stunden ist der Brechungsindex von $n_D^{23}$ : 1.4520 auf $n_D^{23}$ : 1,4606 gestiegen.

B) Abstoppung der Rohlösung

Drei Proben von jeweils 672 g wurden entnommen und sofort mit jeweils 0,5 mmol Katalysatorgift abgestoppt. Die erfindungsgemäßen Katalysatorgifte stabilisierten die Rohlosung wirksam über die Zeit, während der Vergleichsstopper Phosphorsäuredibutylester über die Zeit an Wirksamkeit verlor. Die Überprüfung erfolgte durch Messung des Brechungsindex, der Zeitraum betrug 84 Tage. Einzelheiten sind Tabelle 4 zu entnehmen.

Tabelle 4

|  | Probe a | Probe b | Probe c |
|---|---|---|---|
| Menge | 672 g | 672 g | 672 g |
| Katalysatorgift | Trifluormethan-sulfonsäuretri-methylsilylester | Phosphorsäuretris-(trimethylsilyl-ester) | Phosphorsäuredibutyl-ester |
| $n_D^{23}$: nach Abstoppung | 1,4609 | 1,4608 | 1,4609 |
| $n_D^{23}$: nach 84 Tagen | 1,4610 | 1,4608 | 1,4688 |

**Patentansprüche**

1. Verfahren zur Konditionierung und/oder Reinigung von organischen Isocyanaten, dadurch gekennzeichnet, daß man organische Isocyanate bei 20 bis 150 °C mit 0,001 bis 1 Mol-%, bezogen auf die Menge

11

des Isocyanats, einer silylierten Säure der Formel

X-[Si(CH$_3$)$_3$]$_n$

vermischt und gegebenenfalls die so erhaltene Mischung nach einer Verweildauer von mindestens 5 Minuten einer destillativen Aufarbeitung unterzieht, wobei

X    für den neutralen Säurerest steht, wie er durch Entfernung der aciden Wasserstoffatome aus einer n-basischen Säure mit einem pKa-Wert von maximal 3 erhalten wird, wobei Halogenwasserstoffsäuren ausgenommen sind, und

n    für eine ganze Zahl von 1 bis 3 steht.

2.  Verfahren gemäß Anspruch 1, dadurch gekennzeichnet, daß man als silylierte Sauren der in Anspruch 1 genannten Formel O-silylierte, Sauerstoff enthaltende Säuren verwendet, die in nicht-silylierter Form einen pKa-Wert von maximal 2 aufweisen,

3.  Verfahren gemäß Anspruch 1 und 2, dadurch gekennzeichnet, daß man als silylierte Säuren Trifluormethansulfonsäuretrimethylsilylester oder Phosphorsäuretris(trimethylsilylester) verwendet werden.

4.  Verfahren gemäß Anspruch 1 bis 3, dadurch gekennzeichnet, daß man als organisches Isocyanat 1,6-Diisocyanatohexan, 1-Isocyanato-3,3,5-isocyanatomethyl-cyclohexan, 4,4'-Diisocyanatodicyclohexylmethan oder beliebige Gemische dieser Diisocyanate verwendet.

5.  Verfahren gemäß Anspruch 1 bis 3, dadurch gekennzeichnet, daß man als organisches Isocyanat aromatische Diisocyanate, ausgewählt aus der Gruppe bestehend aus (i) 2,4- und/oder 2,6-Diisocyanatotoluol, (ii) 2,2'-, 2,4'- und/oder 4,4'-Diisocyanatodiphenylmethan und (iii) beliebigen Gemischen dieser Diisocyanate verwendet.

6.  Verfahren gemäß Anspruch 1 bis 3, dadurch gekennzeichnet, daß man als organische Isocyanate Gemische aus (i) 1,6-Diisocyanatohexan und/oder 1-Isocyanato-3,3,5-trimethyl-5-isocyanatomethylcyclohexan und (ii) Isocyanuratgruppen aufweisenden Polyisocyanaten auf Basis dieser Diisocyanate verwendet, wie sie bei der katalytischen Trimerisierung der genannten Diisocyanate anfallen und das so erhaltene Gemisch anschließend unter Gewinnung eines praktisch monomerenfreien, Isocyanuratgruppen aufweisenden Polyisocyanats der destillativen Aufarbeitung unterzieht.

7.  Verfahren gemäß Anspruch 6, dadurch gekennzeichnet, daß man als organische Isocyanate solche Gemische aus den in Anspruch 6 genannten Einzelkomponenten (i) und (ii) verwendet, die unter Verwendung von Ausgangsdiisocyanaten (i) erhalten worden sind, die ihrerseits der Einwirkung von 0,001 bis 1 Mol-%, bezogen auf Diisocyanat, einer silylierten Säure der Formel

X-[Si(CH$_3$)$_3$]$_n$

unter anschließender destillativer Aufarbeitung unterzogen worden waren, wobei X und n die in Anspruch 1 genannte Bedeutung haben.

**Claims**

1.  A process for the conditioning and/or purification of organic isocyanates, characterized in that organic isocyanates are mixed at 20 to 150°C with 0.001 to 1 mol-%, based on the quantity of isocyanate, of a silylated acid corresponding to the following formula

X-[Si(CH$_3$)$_3$]$_n$

in which

X    is the neutral acid residue obtained by removal of the acidic hydrogen atoms from an n-basic acid having a pKa value of at most 3, hydrohalic acids being excluded, and

n    is an integer of 1 to 3,

and the resulting mixture is optionally worked up by distillation after a residence time of at least 5

EP 0 508 216 B1

minutes.

2. A process as claimed in claim 1, characterized in that O-silylated oxygen-containing acids which, in non-silylated form, have a pKa value of at most 2, are used as the silylated acids corresponding to the formula in claim 1.

3. A process as claimed in claims 1 and 2, characterized in that trifluoromethane sulfonic acid trimethyl silyl ester or phosphoric acid tris-(trimethyl silyl ester) is used as the silylated acid.

4. A process as claimed in claims 1 to 3, characterized in that 1,6-diisocyanatohexane, 1-isocyanato-3,3,5-isocyanatomethyl cyclohexane, 4,4'-diisocyanatodicyclohexyl methane or a mixture of these diisocyanates is used as the organic isocyanate.

5. A process as claimed in claims 1 to 3, characterized in that the organic isocyanate is an aromatic diisocyanate selected from the group consisting of (i) 2,4- and/or 2,6-diisocyanatotoluene, (ii) 2,2'-, 2,4'- and/or 4,4'-diisocyanatodiphenyl methane and (iii) mixtures of these diisocyanates.

6. A process as claimed in claims 1 to 3, characterized in that the organic isocyanate is a mixture of (i) 1,6-diisocyanatohexane and/or 1-isocyanato-3,3,5-trimethyl-5-isocyanatomethyl cyclohexane and (ii) isocyanurate-modified polyisocyanates based on these diisocyanates which are formed in the catalytic trimerization of the diisocyanates mentioned and the resulting mixture is subsequently subjected to working up by distillation with recovery of a substantially monomer-free, isocyanurate-modified polyisocyanate.

7. A process as claimed in claim 6, characterized in that the organic isocyanates used are mixtures of the individual components (i) and (ii) mentioned in claim 6 which have been obtained using starting diisocyanates (i) which, in turn, have been subjected to the effect of 0.001 to 1 mol-%, based on diisocyanate, of a silylated acid corresponding to the formula

$X-[Si(CH_3)_3]_n$

in which X and n are as defined in claim 1, with subsequent working up by distillation.

**Revendications**

1. Procédé pour le conditionnement et/ou la purification d'isocyanates organiques, caractérisé en ce qu'on mélange des isocyanates organiques à une température de 20 à 150°C avec de 0,001 à 1 mole %, rapportée à la quantité de l'isocyanate d'un acide silylé répondant à la formule

$X-[Si(CH_3)_3]_n$

et on soumet éventuellement le mélange ainsi obtenu après un temps de séjour d'au moins 5 minutes, à un traitement par distillation,
dans laquelle
    X     représente le radical d'acide neutre tel qu'on l'obtient par élimination des atomes d'hydrogène acides d'un acide n basique ayant une valeur pKa de maximum 3, des acides halogénhydriques étant exclus, et
    n     représente un entier de 1 à 3.

2. Procédé selon la revendication 1, caractérisé en ce que, comme acides silylés répondant à la formule mentionnée à la revendication 1, on utilise des acides O-silylés contenant de l'oxygène, qui présentent une valeur pKa de maximum 2 sous la forme non silylée.

3. Procédé selon les revendications 1 et 2, caractérisé en ce que, comme acides silylés, on utilise l'ester triméthylsilylique de l'acide trifluorométhanesulfonique ou le tris-(ester triméthylsilylique) de l'acide phosphorique.

13

4. Procédé selon les revendications 1 à 3, caractérisé en ce que, comme isocyanate organique, on utilise le 1,6-diisocyanatohexane, le 1-isocyanato-3,3,5-isocyanatométhylcyclohexane, le 4,4'-diisocyanatodicyclohexylméthane ou n'importe quels mélanges de ces diisocyanates.

5. Procédé selon les revendications 1 à 3, caractérisé en ce que, comme isocyanate organique, on utilise des diisocyanates aromatiques choisis parmi le groupe constitué par (i) le 2,4- et/ou 2,6-diisocyanatotoluène, (ii) le 2,2'-, 2,4'- et/ou 4,4'-diisocyanatodiphénylméthane et (iii) n'importe quels mélanges de ces diisocyanates.

6. Procédé selon les revendications 1 à 3, caractérisé en ce que, comme isocyanates organiques, on utilise des mélanges constitués par (i) le 1,6-diisocyanatohexane et/ou le 1-isocyanato-3,3,5-triméthyl-5-isocyanatométhylcyclohexane et (ii) des polyisocyanates présentant des groupes isocyanurate à base de ces diisocyanates, tels qu'on les obtient lors de la trimérisation catalytique des diisocyanates mentionnés, et on soumet ensuite le mélange ainsi obtenu au traitement par distillation en obtenant un polyisocyanate pratiquement exempt de monomères, présentant des groupes isocyanurate.

7. Procédé selon la revendication 6, caractérisé en ce que, comme isocyanates organiques, on utilise des mélanges constitués par les composants individuels (i) et (ii) mentionnés à la revendication 6, que l'on obtient en utilisant des diisocyanates (i) de départ qui ont été soumis à leur tour à l'action de 0,001 à 1 mole %, rapportée au diisocyanate d'un acide silylé répondant à la formule

$$X\text{-}[Si(CH_3)_3]_n$$

avec traitement ultérieur par distillation, X et n ayant la signification mentionnée à la revendication 1.